(19) Europäisches Patentamt
European Patent Office
Office européen des brevets

(11) Numéro de publication: **0 304 381 B1**

(12) **FASCICULE DE BREVET EUROPEEN**

(45) Date de publication du fascicule du brevet: **27.12.90**

(51) Int. Cl.⁵: **C09J 7/02,** A61L 15/26

(21) Numéro de dépôt: **88420275.5**

(22) Date de dépôt: **04.08.88**

(54) **Article stratifié souple pour adhésif de transfert.**

(30) Priorité: **14.08.87 FR 8711697**

(43) Date de publication de la demande: **22.02.89 Bulletin 89/8**

(45) Mention de la délivrance du brevet: **27.12.90 Bulletin 90/52**

(84) Etats contractants désignés: **AT BE CH DE ES FR GB GR IT LI LU NL SE**

(56) Documents cités:
**EP-A- 0 033 193**
**EP-A- 0 180 377**
**EP-A- 0 252 858**
**GB-A- 2 073 765**
**US-A- 4 460 371**

(73) Titulaire: **RHONE-POULENC CHIMIE, 25, quai Paul Doumer, F-92408 Courbevoie Cédex(FR)**

(72) Inventeur: **Cavezzan, Jacques, Résidence du Parc Tête d'Or 4 Allée Marchel Achard, F-69100 Villeurbanne(FR)**
Inventeur: **Di Martino, Jean-Louis, Route de Lieu Dieu Chatonnay, F-38540 Saint-Jean-de-Bournay(FR)**

(74) Mandataire: **Trolliet, Maurice et al, RHONE-POULENC CHIMIE Service Brevets Chimie Centre de Recherches des Carrières B.P. 62, F-69192 Saint-Fons Cédex(FR)**

**EP 0 304 381 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des compositions de polysiloxanes particulièrement appropriées à la réalisation de revêtements pelables, et tout particulièrement utiles pour des adhésifs de transfert dont la couche d'adhésif et la couche anti-adhésive sont toutes les deux en silicones...

Les revêtements anti-adhérents sont utiles toutes les fois qu'il est nécessaire d'obtenir une surface ou un matériau qui soit relativement non-adhérent à d'autres matériaux auxquels il adhèrerait normalement. On utilise largement les compositions de silicones anti-adhérentes pour papiers comme revêtements s'enlevant d'adhésifs de contact pour des étiquettes, des stratifiés décoratifs, des rubans d'adhésifs de transfert, etc... Les revêtements anti-adhérents de silicone pour papier, polyéthylène, Mylar et autres substrats de ce type sont également utiles comme surfaces non collantes pour des applications de manutention de produits alimentaires et d'emballage industriel. On trouve ordinairement ces revêtements sous une forme dépourvue de solvant, sous la forme d'une solution dans un solvant, ou sous la forme d'une émulsion aqueuse.

Lorsque des étiquettes sont revêtues d'un adhésif, il est souhaitable que le papier placé au dos puisse être retiré facilement de l'étiquette lorsqu'elle doit être utilisée, sans que la qualité d'adhésivité de l'étiquette soit altérée par sa séparation du substrat sur lequel elle était stockée. Le même principe s'applique à certains types de rubans adhésifs en rouleaux. Il est nécessaire que le ruban se déroule facilement tout en conservant ses propriétés adhésives. On peut y parvenir en recouvrant le côté non adhésif du ruban avec une composition de silicone anti-adhérente pelable qui viendra au contact de l'adhésif lors de la fabrication du rouleau de ruban.

On peut utiliser les revêtements anti-adhérents en liaison avec des adhésifs à base de silicone pour former un système d'adhésifs de transfert. Ce type d'adhésif peut avoir de nombreuses applications comme par exemple, la restauration d'oeuvres d'art, comme cela est décrit en détail dans le brevet français FR-A-2 478 657 et le brevet européen EP-A-33 193. Dans ce type d'adhésif, on recouvre un premier substrat, qui peut ordinairement être constitué par n'importe quel type de film non poreux ou de papier glacé, et de préférence par du papier kraft supercalendré, avec un revêtement de silicone anti-adhérent, qui peut être retiré d'un adhésif de contact agressif à base de silicone. On décrira plus tard ces deux compositions dans la présente demande. On fait durcir le revêtement anti-adhérent sur le premier substrat par des moyens bien connus, et ordinairement par la chaleur. On applique ensuite l'adhésif de contact à base de silicone sur le revêtement anti-adhérent et on le fait durcir sur place, de sorte que le premier substrat porte maintenant deux revêtements, à savoir le revêtement anti-adhérent de silicone et l'adhésif de silicone.

On applique ensuite le premier substrat portant le double revêtement sur un second substrat approprié au renforcement et à la conservation de l'oeuvre d'art de manière à former un stratifié. Lorsque cette étape de formation de stratifié est terminée, on dispose d'un système d'adhésif de transfert que l'on pourra utiliser pour renforcer l'oeuvre d'art à n'importe quel moment futur voulu. On procède à la restauration en séparant le premier substrat et son revêtement anti-adhérent du second substrat qui conserve l'adhésif de contact à base de silicone, puisque l'adhésif agressif adhère beaucoup plus au second substrat qu'au revêtement anti-adhérent avec lequel il était en contact. L'adhésif de silicone a donc été tranféré du substrat portant le revêtement anti-adhérent et on se trouve en présence d'un second substrat renforçateur sur l'oeuvre d'art pour former un stratifié.

Ce type de système d'adhésif de transfert ne se limite bien évidemment pas à la restauration de peintures, mais on peut l'utiliser toutes les fois qu'il est souhaitable d'appliquer un adhésif de contact complètement durci sur pratiquement n'importe quelle sorte de substrat. Une fois que l'adhésif a été transféré, il est immédiatement prêt à l'emploi si on applique des pressions modérées.

Ces systèmes d'adhésifs de transfert, à base de silicone présentent encore l'avantage de rester souples dans une gamme étendue de températures. On ne trouve pas cette souplesse pour des adhésifs organiques comme des matières acryliques ou des résines époxy qui constituent des adhésifs inefficaces lorsqu'on les applique sur un substrat qui atteint ordinairement des températures relativement élevées ou basses. Par exemple, un système d'adhésif de transfert à base de silicone à teneur élevée en groupes phényles sera particulièrement utile, si on souhaite appliquer un adhésif sur un moteur. Les adhésifs organiques s'écailleront, se fendilleront ou n'adhèreront pas d'une manière satisfaisante à ce type de substrat. On peut toutefois déposer l'adhésif de silicone sur son substrat de transfert portant un revêtement anti-adhérent et l'y faire durcir, après quoi on peut le transférer plus tard sur la partie chaude du moteur, qui conservera l'adhésif. Un adhésif de contact à base de silicone est capable de supporter les températures normalement élevées auxquelles un moteur peut être soumis. On peut ensuite appliquer une étiquette, ou un autre stratifié sur cet adhésif.

Dans ce type d'adhésif de transfert le problème technique est extrêmement difficile à résoudre.

Il est nécessaire en effet que le revêtement anti-adhérent et l'adhésif adhèrent suffisamment entre eux pour assurer la cohérence du stratifié. Bien entendu cette adhésivité doit rester modeste pour que l'adhésif puisse être facilement et totalement transféré lors de l'utilisation du stratifié. En outre cet adhésif, une fois séparé de la couche anti-adhérente silicone doit adhérer suffisamment sur le substrat sur lequel il est transféré.

Ce substrat peut être quelconque et être en particulier un élastomère silicone contenant un médicament dans le cas de l'administration transdermique d'un médicament. L'élastomère silicone ainsi adhésivé doit présenter une bonne adhésivité sur la peau du patient.

La présente invention propose une solution technique à ce problème consistant en un choix spécifique à la fois d'une composition silicone anti-adhérente et d'un adhésif silicone.

De façon surprenante et inattendue, contrairement à l'enseignement de FR-A-2 478 657 et de EP-A-33 193 précités, on a pu mettre en évidence, conformément à la présente invention que:
- la composition organopolysiloxane anti-adhérente doit être utilisée en solution dans un solvant organique, être exempte de résine silicone MQ et présenter des ingrédients spécifiques,
- l'adhésif silicone phénylé ne doit pas comporter de peroxydes organiques.

La présente invention concerne en effet un article stratifié souple comportant :
- un premier substrat comportant sur au moins une face une couche anti-adhérente silicone provenant de la réticulation d'une composition organopolysiloxane comportant :

a) - au moins un organopolysiloxane comportant par molécule au moins deux groupes SiVi,
b) - au moins un organohydrogénopolysiloxane comportant par molécule au moins trois groupes SiH,
c) - une quantité catalytiquement efficace d'un catalyseur d'hydrosilylation qui est un composé d'un métal du groupe du platine, caractérisé en ce que entre 0,5 et 60 %, de préférence entre 1,5 et 20 % en nombre des groupes SiVi de la composition sont apportés par un cyclotrisiloxane vinylé de formule :

$R(CH_2 = CH)SiO_3 (1)$

dans laquelle R est choisi parmi un radical alkyle en $C_1$-$C_4$, un radical phényle et un radical trifluoro-3,3,3 propyle, de préférence R est un radical méthyle,
d) - un solvant organique représentant 30 à 99 % en poids du poids total de la solution.
- une couche d'adhésif appliquée sur la couche anti-adhérente, l'adhésif étant un organopolysiloxane exempt de peroxyde organique et qui est le produit d'intercondensation d'une résine MQ et d'une gomme polyalkylphénylsiloxane.
- un deuxième substrat appliqué sur l'adhésif.

Dans ce qui suit ou ce qui précède, sauf mentions contraires, les pourcentages et parties sont en poids.

La composition organopolysiloxane anti-adhérente est décrite en détail dans la demande de brevet français 86/09 311 déposée le 24 juin 1986.

Les organopolysiloxanes (A) de base sont des diorganopolysiloxanes de formule :

$R_2R'\,SiO(SiR_2O)_n(SiRR'O)_mSiR_2R' (2)$

dans laquelle n et m sont des nombres entiers pouvant être nuls séparemment et dont la somme a une valeur telle que l'organopolysiloxane a une viscosité à 25 °C d'au moins 20 mPa.s et d'au plus 30 000 000 mPa.s, les radicaux R identiques ou différents ont la signification donnée ci-dessus à la formule (1) et R' est R ou un radical vinyle étant entendu que si m = O R' est un radical vinyle.

Les organopolysiloxanes (A) peuvent comporter également de préférence les diorganocyclosiloxanes de formule :

$R(CH_2 = CH)SiO\ _p (3)$

dans laquelle R a la même signification que ci-dessus et p est un nombre entier compris entre 4 et 10 inclus.

Les organopolysiloxanes (A) de formules (2) et (3) sont bien connus et sont décrits notamment dans les brevets américains US-A-3 220 972, US-A-3 344 111 et US-A-3 436 366, ces brevets décrivant également les organohydrogénopolysiloxanes (B).

De préférence au moins 60 % molaire des radicaux R sont des radicaux méthyle, et, de façon plus préférée tous les radicaux R sont des radicaux méthyle.

L'organohydrogénopolysiloxane (B) peut être linéaire, cyclique ou ramifié.

L'organohydrogénopolysiloxane (B) sensiblement linéaire ou ramifié possède des motifs de formule générale moyenne :

$$R_xH_ySiO_{\frac{4-x-y}{2}} \qquad (4)$$

dans laquelle les radicaux R identiques ou différents ont la signification indiquée plus haut, mais la possibilité pour certains radicaux R de représenter des radicaux vinyle n'étant pas exclue. De préférence au moins 80 % des radicaux R sont des radicaux méthyle.

Le symbole x représente un nombre quelconque allant de 1 à 1,99, le symbole y représente un nombre quelconque allant de 0,1 à 1, la somme x + y allant de 1,7 à 2,6. De préférence sont utilisés comme organohydrogénopolysiloxanes (B) des méthylhydrogénopolyxiloanes.

Les organohydrogénopolysiloxanes (B) sont disponibles sur le marché des silicones, de plus leurs techniques de préparation sont maintenant bien au point. L'une des techniques les plus utilisées consiste, dans un premier temps, à cohydrolyser des mélanges appropriés constitués de chorosilanes choisis parmi ceux de formules : $R_3SiCl$, $R_2SiCl_2$, $RSiCl_3$, $SiCl_4$, $HR_2SiCl$, $HRSiCl_2$, $HSiCl_3$. Par mélanges appropriés doivent être compris des mélanges qui renferment chacun, par atome de silicium, un nombre de radicaux R et un nombre d'atomes d'hydrogène coïncidant respectivement avec les valeurs représentées par les symboles x et y de la formule générale moyenne, la somme de ces nombres devant également coïncider avec les valeurs permises pour la somme x + y.

Dans un deuxième temps les cohydrolysats sont portés à une température allant de 80 à 220 °C, de préférence en présence d'agents acides tels que l'acide sulfurique, les terres activées par un acide. Lors de ce chauffage il se produit un réarrangement des liaisons siloxaniques ainsi que la condensation des groupes SiOH. Ces transformations conduisent aux polymères organohydrogénoplysiloxaniques (B) qui possèdent ainsi, en fonction des mélanges de chlorosilanes de départ, des tructures linéaires, cycliques ou ramifiées.

Généralement les constituants (A) et (B) se trouvent dans la composition en quantité telle que le rapport en nombre des groupes SiH sur les groupes SiVi soit compris entre 0,5 et 5, de préférence entre 0,6 et 2,5.

Dans les applications de revêtement et plus particulièrement dans les applications pour l'anti-adhérence du papier, on peut utiliser pour 100 parties en poids de polymère (A) de 0,1 à 50 parties en poids de polymère (B).

On peut utiliser comme catalyseur (C) des composés d'un métal du groupe du platine, en particulier leurs sels et complexes notamment les complexes de platine-oléfine comme décrits dans les brevets US-A-3 159 601 et US-A-3 159 662, les produits de réaction des dérivés du platine avec des alcools des aldéhydes et des éthers décrits dans le brevet US-A-3 220 972, les catalyseurs platine-vinylsiloxane décrits dans les brevets français FR-A-1 480 409 ainsi que les complexes décrits dans les brevets US-A-3 715 334, US-A-3 775 452 et US-A-3 814 730, ainsi qu'un catalyseur au rhodium tel que décrit dans les brevets US-A-3 296 291 et US-A-3 928 629.

Les métaux préférés du groupe du platine sont le platine et le rhodium ; le ruthénium bien que moins actif mais moins cher, est églament utilisable.

On utilise généralement de 5 à 200 ppm de catalyseur calculé en poids de métal par rapport au poids des polysiloxanes a SiVi et à SiH.

Les compositions antiadhérentes selon l'invention doivent être en solution dans un solvant organique volatil, choisi par exemple parmi les alcanes, les coupes pétrolières renfermant des composés paraffiniques, le toluène, l'heptane, le xylène, l'isopropanol, la méthylisobutylcétone, le tétrahydrofuranne, le chlorobenzène, le chloroforme, le 1,1,1-trichloroéthane, les dérivés du monoéthylèneglycol et de méthylèneglycol.

De préférence, le solvant forme au moment de l'emploi de 50 à 99% en poids de la solution.

Après évaporation du solvant, la composition antiadhérente durcit à température ambiante ou plus rapidement par chauffage. Ces solutions sont donc utiles comme compositions de revêtement anti-adhérentes pour des supports souples en métal, papier, matière plastique, carton, etc .... pouvant constituer le premier substrat du stratifié selon l'invention.

Pour rendre le premier substrat non-adhérent on applique une quantité de composition conforme à l'invention comprise généralement entre 0,1 et 10 g par $m^2$ de surface à enduire et à réticuler thermiquement la composition.

Les compositions peuvent être appliquées à l'aide des dispositifs utilisés sur les machines industrielles d'enduction du papier telles que le cylindre gravé "Mille points", le systène nommé "Reverse Roll". Une fois déposées sur les supports, les compositions sont durcies par apport d'énergie, dont au moins une partie peut être fournie par un rayonnement U.V., en quelques secondes par circulation sous une lumière U.V. et dans des fours-tunnels chauffés vers 60-200 °C; le temps de passage dans ces fours varie généralement de 2 à 30 secondes. Il est fonction, pour une longueur donnée des fours, de la vitesse à laquelle circulent les supports enduits (cette vitesse peut dépasser 200 mètres par minute).

Les quantités de compositions déposées sur les supports sont variables et s'échelonnent le plus souvent entre 0,1 et 10 g/$m^2$ de surface traitée. Ces quantités dépendent de la nature des supports et des propriétés anti-adhérentes recherchées. Elles sont le plus souvent comprises entre 0,5 et 1,5 g/$m^2$ pour des supports non poreux.

Les produits de formule (1) sont des produits bien connus dont les procédés de préparation sont en particulier décrits dans les brevets américains US-A-3 607 898, US-A-3 763 212, US-A-3 989 733 et la demande de brevet japonais publiée KOKAI 74/124 067, cités comme références. On utilise préférentiellement le triméthyltrivinylcyclotrisiloxane.

Les produits de formule (1) conduisent à des revêtements plus durs bien que suffisamment souples ce qui facilite grandement la découpe automatique en machine des complexes souples, en particulier des papiers, sans altération de l'effet anti-adhérent.

Quant à l'adhésif phénylé, autre objet de l'invention, il résulte généralement de l'intercondensation d'un mélange d'une résine de polyorganosiloxane et d'au moins une gomme de polyalkylarylsiloxane. On

utilise un adhésif particulier comprenant 100 parties en poids d'une gomme de polyalkylphénylsiloxane à terminaisons silanol présentant une viscosité comprise entre 1 et 200 millions de centipoises à 25 °C et une teneur en motifs organophénylsiloxy comprise entre 5 et 40 moles pour cent moles de motifs constituant la gomme. On recommande particulièrement d'utiliser une gomme de polydiméthyldiphénylsiloxane à terminaisons silanol présentant une teneur en groupes diphénylsiloxy comprise entre 5 et 25 moles pour cent et une viscosité comprise entre 20 et 80 x $10^{+6}$ mPa.s à 25 °C. On combine cette gomme avec de 50 à 200 parties en poids d'une résine de silicone MQ telle que décrite par exemple dans le brevet US-A-2 676 182, présentant un rapport de M à Q compris entre 0,5 et 1,0.

On peut par exemple combiner 100 parties de la gomme précédente avec de 100 à 150 parties de résine MQ, sachant que M représente $(CH_3)_3SiO_{1/2}$ et Q représente $(SiO_2)$.

Cette intercondensation, effectuée avant l'enduction de l'adhésif, se fait de préférence par chauffage à 80-120 °C du mélange de la gomme et de la résine MQ en présence de catalyseurs basiques tels que NaOH, KOH, silanolate de potassium.

Des adhésifs phénylés convenables sont par exemples décrits dans le brevet américain us-A2 857 356.

La fabrication du stratifié souple à adhésif de transfert à base de silicone comprend les étapes de dépôt et de durcissement du revêtement anti-adhérent sur un premier substrat qui sera ordinairement en papier ou une pellicule en polymère organique. Le papier peut se trouver sous la forme de feuilles ou de rouleaux, etc .....Il faut revêtir au moins une des surfaces du papier ou d'un autre substrat. Toutefois, si on revêt les deux côtés, on pourra enrouler le papier doublement revêtu à la manière d'un ruban.

On dépose ensuite sur le revêtement anti-adhérent un revêtement d'adhésif de contact à base de silicone que l'on fait ensuite prédurcir ou préréticuler par des procédés bien connus sur le revêtement anti-adhérent généralement par chauffage à 60-150 °C avec élimination du solvant.

Lorsque l'on fait durcir l'adhésif de silicone sur le revêtement anti-adhérent, il est prêt à l'emploi, ou on peut le stocker pour une utilisation ultérieure.

Le premier substrat est maintenant doublement revêtu du revêtement anti-adhérent et du film adhésif. On peut alors appliquer le substrat doublement revêtu sur un second substrat que l'on veut rendre adhésif. Ce second substrat peut être un élastomère silicone. On sépare ou on détache ensuite le premier substrat portant le revêtement anti-adhérent du second substrat, ce qui a pour effet de transférer le film d'adhésif sur le substrat. Le second substrat est maintenant devenu adhésif et on peut l'appliquer sur un troisième substrat, qui peut être par exemple la peau d'un patient dans le cas où le second substrat est un élastomère silicone (administration transdermique de médicament). Dans ce dernier cas l'élastomère silicone contenant le médicament provient de la réticulation d'une composition organopolysiloxane adaptée à la libération du médicament vers la peau du patient. De telles compositions organopolysiloxanes sont connues. Elles sont par exemple décrites dans le brevet US-A-4 331 651.

Il faut, bien évidemment, comprendre que le second substrat peut être constitué par la pièce que l'on souhaite rendre adhésive immédiatement, ou par une surface portant un revêtement anti-adhérent comme le verso d'un ruban support d'adhésif.

- EXEMPLE 1 :

1. - Préparation de la composition silicone anti-adhérente :

On homogénéise le mélange suivant :

* 300 parties d'une gomme polyvinylméthyldiméthylsiloxane ayant des motifs vinyle dans la chaîne et à terminaison triméthylsiloxyle ayant environ 0,07 % en poids de groupements vinyle et présentant une viscosité d'environ $20.10^6$ mPa.s à 25 °C,
* 7 parties de tétraméthyl-1,3,5,7 tétravinyl-1,3,5,7 cyclotétrasiloxane ($D_4Vi$),
* 8 parties d'un copolymère hydrogénométhyldiméthylpolysiloxane comportant des motifs méthylhydrogénosiloxyle et diméthylsiloxyle et à terminaison triméthylsiloxyle ayant environ 1 % en poids d'atomes d'hydrogène liés au silicium et une viscosité d'environ 45 mPa.s à 25 °C,
* 685 parties de toluène.

On ajoute à ce bain :

* 1 partie de triméthyltrivinylcyclotrisiloxane ($D_3Vi$),
* 60 ppm de platine ($3.10^{-4}$ at.g. de Pt/kg de composition) sous la forme d'un complexe du platine préparé à partir de l'acide chloroplatinique et de divinyl-1,3 tétraméthyl-1,1,3,3 disiloxane comme décrit à l'exemple 3 du brevet US-A-3 814 730.

On agite vigoureusement, à température ambiante, le mélange pendant quelques minutes et on dépose ensuite ce mélange (environ de 1 à 3 g/m²) sur une pelicule de polyester TERPHANE r au moyen d'une barre d'enduction et on fait durcir pendant 10 secondes la composition silicone dans un four à circulation d'air forcé réglé à 120 °C.

2. - Préparation de l'adhésif silicone :

Résine MQ : cette résine est constituée de motifs M : $(CH_3)_3$ $SiO_{0,5}$ et Q : $SiO_2$ répartis suivant un rapport molaire M/Q de 0,6 ; elle renferme 3,4 % de radicaux OH, sa Mn est de l'ordre de 5 000.

Gomme phénylée : cette gomme est un polydiméthyldiphénylsiloxane à extrémités silanol de viscosité 30 millions mPas.s à 25 °C et ayant une teneur en motifs diphénylsiloxy de 7 % en poids.

Préparation de l'adhésif : on homogénéise un mélange de 21,7 parties de gomme, 33,3 parties de résine MQ et du xylène.

On chauffe le mélange à 90 °C pendant 2 heures : en présence de 40 parties de potasse par millions de parties de gomme + résine MQ, et on obtient un adhésif à 55 % d'extrait sec présentant une viscosité de 60 000 mPa.s à 25 °C.

Sur la pellicule therphane enduite de l'antiadhérent silicone, on dépose au râcle, sur l'antiadhérent, l'adhésif silicone à raison de 60 g d'adhésif par $m^2$.

On évapore le xylène de l'adhésif par chauffage du ruban pendant 2 minutes à 100 °C.

On contrecolle la pellicule de terphane adhésivée sur un ruban d'élastomère silicone polyaddition, commercialisé par la Société RHONE-POULENC sous la dénomination commerciale RTV 141 r.

L'ensemble du stratifié est soumis, à température ambiante, à une pression de 4,5 MPa.

Le stratifié obtenu est laissé à température ambiante pendant 48 heures et on mesure les forces F de décollement en g/cm suivant la norme européenne AFERA 4001.

On obtient un transfert complet et sans défaut de l'adhésif sur l'élastomère silicone et une force de décollement comprise entre 100 et 150 g/cm.

- EXEMPLE COMPARATIF 2 :

On procède exactement comme à l'exemple 1, sauf que l'antiadhérent comporte pour 100 parties d'extrait sec, 3,5 parties de résine MQ utilisée pour l'adhésif. On observe un ralentissement de la vitesse de polymérisation de la couche antiadhérente et le transfert de l'adhésif est de mauvaise qualité.

- EXEMPLE COMPARATIF 3 :

On procède exactement comme à l'exemple 1 sauf que l'adhésif comporte 2 parties de peroxyde de 2,4-dichlorobenzoyle pour 100 parties d'extrait sec d'adhésif.

Le transfert d'adhésif est inexistant après décollement..

- EXEMPLE COMPARATIF 4 :

On procède exactement comme à l'exemple comparatif 2 sauf que l'adhésif contient en outre du peroxyde dans les mêmes conditions qu'à l'exemple comparatif 3.

On n'a pas réussi à transférer l'adhésif sur l'élastomère silicone après décollement.

- EXEMPLE 5:

On procède exactement de la même façon qu'à l'exemple 1 sauf que la composition antiadhérente à catalyser, a la composition suivante :

* 350 parties de la gomme vinylée utilisée à l'exemple 1,
* 150 parties d'une huile diméthylpolysiloxane bloquée à chacune de ses extrémités par des motifs diméthylvinylsiloxy, de viscosité 1 000 mPa.s à 25 °C ayant une teneur pondérale en radicaux vinyle liés aux atomes de silicium de 0,3 %,
* 7 parties de $D_4Vi$,
* 8 parties du même copolymère hydrogénométhyldiméthylpolysiloxane utilisé à l'exemple 1,
* 485 parties de toluène.

On contrecolle la pellicule de terphane adhésivée sur un papier couché en polyéthylène à la place de l'élastomère silicone.

On obtient un transfert complet et sans défaut de l'adhésif et une force de décollement de 170 g/cm.

**Revendications**

1. - Article stratifié souple comportant :

- un premier substrat comportant sur au moins une face une couche anti-adhérente silicone provenant de la réticulation d'une composition organopolysiloxane comportant :

a) - au moins un organopolysiloxane comportant par molécule au moins deux groupes SiVi,

b) - au moins un organohydrogénopolysiloxane comportant par molécule au moins trois groupes SiH,

c) - une quantité catalytiquement efficace d'un catalyseur d'hydrosilylation qui est un composé d'un métal du groupe du platine, caractérisé en ce que entre 0,5 et 60%, de préférence entre 1,5 et 20 % en

nombre des groupes SiV de la composition sont apportés par un cyclotrisiloxane vinylé de formule :

$R(CH_2 = CH)SiO_3(1)$

dans laquelle R est choisi parmi un radical alkyle en $C_1$-$C_4$, un radical phényle et un radical trifluoro-3,3,3 propyle, de préférence R est un radical méthyle,
d) - un solvant organique représentant 50 à 99 % en poids du poids total de la solution.
- une couche d'adhésif appliquée sur la couche anti-adhérente, l'adhésif étant un organopolysiloxane exempt de peroxyde organique et qui est le produit d'intercondensation d'une résine MQ et d'une gomme polyalkylphénylsiloxane.
- en deuxième substrat appliqué sur l'adhésif.

2. -Article selon la revendication 1, caractérisé en ce que le deuxième substrat est un élastomère silicone.

3. - Article selon la revendication 2, caractérisé en ce que l'élastomère silicone contient un médicament.

4. - Procédé de préparation d'un article stratifié tel que défini à l'une quelconque des revendications précédentes, caractérisé en ce qu'on dépose et on durcit le revêtement antiadhérent sur le premier substrat, on dépose sur le revêtement antiadhérent durci l'adhésif silicone que l'on prériticule puis on applique le substrat doublement revêtu sur le second substrat que l'on veut rendre adhésif.

## Claims

1. A flexible laminated article comprising:
– a first substrate comprising on at least one face an anti-adherent silicone layer originating from the crosslinking of an organopolysiloxane composition comprising:
a) at least one organopolysiloxane comprising at least two SiVi groups per molecule,
b) at least one organohydrogenopolysiloxane comprising at least three SiH groups per molecule,
c) a catalytically effective amount of a hydrosilylation catalyst which is a compound of a metal of the platinum group, characterized in that between 0.5 and 60%, and preferably between 1.5 and 20%, by number of the SiVi groups of the composition are supplied by a vinyl-cyclotrisiloxane of formula:
$R(CH_2 = CH)SiO_3$ (1)
in which R is chosen from a $C_1$–$C_4$ alkyl radical, a phenyl radical and a 3,3,3-trifluoropropyl radical; preferably R is a methyl radical,
d) an organic solvent representing 50 to 99% by weight of the total weight of the solution,
– an adhesive layer applied on the anti-adherent layer, the adhesive being an organopolysiloxane free from organic peroxide and which is the product of intercondensation of a resin MQ and a polyalkylphenylsiloxane gum, and
– a second substrate applied on the adhesive.

2. Article according to Claim 1, characterized in that the second substrate is a silicone elastomer.

3. Article according to Claim 2, characterized in that the silicone elastomer contains a medicament.

4. Process for the preparation of a laminated article as defined in any one of the preceding claims, characterized in that the anti-adherent coating is deposited and hardened on the first substrate, the silicone adhesive, which is pre-crosslinked, is deposited on the hardened anti-adherent coating and the substrate provided with two coatings is then applied to the second substrate which it is desired to render adhesive.

## Patentansprüche

1. Biegsames Schichtstofferzeugnis, enthaltend:
– ein erstes Substrat, von dem mindestens eine Fläche eine Silikonantihaftschicht aufweist, die von der Vernetzung einer Organopolysiloxanzusammensetzung stammt, enthaltend:
a) mindestens ein Organopolysiloxan, das pro Molekül mindestens zwei Gruppen SiVi aufweist:
b) mindestens ein Organohydrogenpolysiloxan, das pro Molekül mindestens 3 Gruppen SiH aufweist,
c) eine katalytisch wirksame Menge eines Hydrosilylierungskatalysators, der eine Verbindung eines Metalls der Platingruppe ist, dadurch gekennzeichnet, daß zwischen 0,5 und 60%, vorzugsweise zwischen 1,5 und 20% der Anzahl der Gruppen SiVi der Zusammensetzung eingebracht sind durch ein Vinylcyclotrisiloxan der Formel
$R(CH_2 = CH)SiO_3$ (1)
worin R ausgewählt ist unter einem $C_1$–$C_4$-Alkylrest, einem Phenylrest und einem 3,3,3-Trifluorpropylrest, vorzugsweise ist R ein Methylrest,
d) ein organisches Lösungsmittel, das 50 bis 99 Gew.-% des Gesamtgewichts der Lösung darstellt;
– eine Klebstoffschicht, welche auf die Antihaftschicht aufgebracht ist, wobei der Klebstoff ein Organopolysiloxan frei von organischem Peroxid ist und das Produkt der Kondensation zwischen einem Harz MQ und einem Polyalkylphenylsiloxangummi ist;
– ein zweites Substrat, das auf den Klebstoff aufgebracht ist.

2. Erzeugnis gemäß Anspruch 1, dadurch gekennzeichnet, daß das zweite Substrat ein Silikonelastomeres ist.

3. Erzeugnis gemäß Anspruch 2, dadurch gekennzeichnet, daß das Silikonelastomere ein Arzneimittel enthält.

4. Verfahren zur Herstellung eines Schichtstofferzeugnisses, wie es in einem der vorhergehenden Ansprüche definiert ist, dadurch gekennzeichnet, daß man den Antihaftüberzug auf dem ersten Substrat ablagert und härtet, auf dem gehärteten Antihaftüberzug den Silikonklebstoff absetzt, den man vorvernetzt und dann das doppelt bedeckte Substrat auf das zweite Substrat aufbringt, das man klebend machen möchte.